# EUROPEAN PATENT APPLICATION

(11) **EP 1 253 205 A1**
(43) Date of publication of application: **30.10.2002**
(21) Application number: 01109971.0
(22) Date of filing: 24.04.2001
(51) Int. Cl.: C12Q 1/68, C12N 15/10

(54) **Method of blocking amplification of selected sequences**

(71) Applicant: LION Bioscience AG, 69120 Heidelberg (DE)
(72) Inventor: Höfer, Michael, Dr., 69121 Heidelberg (DE); Kranz, Harald, Dr., 69221 Dossenheim (DE); Klink, Martin, 63454 Hanau (DE)
(74) Representative: Schohe, Stefan

(57) **Abstract**

The invention relates to a method for the preferential nucleic acid synthesis reaction of one or more selected regions of one or more target nucleic acids form a group of at least two different target nucleic acids comprising the steps of combining in a reaction mixture necessary reagents, additionally, adding at least two different target nucleic acid molecules together with at least one blocking agent, which is capable of binding at least one of the nucleic acid template molecules in a sequence specific manner, in such a way that the polymerase is not able to utilize the bound target nucleic acid molecule as a template and, exposing the reaction mixture to a temperature at which nucleic acids may be synthesized by the polymerase. Additionally, the invention relates to uses of the method, such as for creating mRNA or other clone libraries as well as kits for performing the method.

## Description

### BACKGROUND OF THE INVENTION

Amplification of nucleic acids is often performed on mixtures of more than one template. Such amplifications are often done using genomic DNA from various sources but also using mRNA from various sources such as different tissues. It is often the case that in such mixtures one or more templates are over represented. One example is the heart tissue in which the actin gene is found to be highly expressed, in most tissues, in human one may find the hemoglobin gene to be expressed highly, in pancreas one will find trypsin protein copies and in liver tissue, *e.g.* lipases

When amplifying nucleic acids such abundant copies are detrimental to the effectiveness of the reaction in general and in particular to the chance of amplifying poorly expressed gene copies or otherwise rare nucleic acids.

For example, it is difficult or nearly impossible to amplify copies of nuclear integrations of mitochondrial DNA from human DNA sources as, the very abundant mitochondrial genome, which is present in great abundance, i.e. at a ratio of 1 to 1000 when compared to the abundance of nuclear integrations per cell, will outcompete the amplification of the integration. Thus, when trying to amplify an integration one will obtain primarily or only product from the mtDNA template.

In a typical human cell about 5-10 particular transcripts make up about 20% of the complete mRNA present, an additional 500-2000 transcripts make up 40-60%, and the remaining percentage of is the very heterogeneous group of up to 20,000 rare transcripts.

The prior art has found a number of solutions for this problem most of which are based on hybridization.

In one particular case the Riken Institute has found a solution in which abundant transcripts are of mRNA are hybridized to a biotinylated RNA which is extracted by use of magnetic particles which are coated with streptavidin (Carninci P, Shibata Y, Hayatsu N, Sugahara Y, Shibata K, Itoh M, Konno H, Okazaki Y, Muramatsu M, Hayashizaki Y, Normalization and subtraction of cap-trapper-selected cDNAs to prepare full-length cDNA libraries for rapid discovery of new genes, Genome Res. 2000 Oct;10(10):1617-30). The drawback is that great amounts of starting material must be available. Another problem is that the hybridization process may result in the extraction of desired rare transcripts due to cross-hybridization. Additionally, the process is expensive as the beads are expensive.

It is thus an objective of this invention to provide for a method that enables the preferential nucleic acid synthesis reaction of one or more selected regions of one or more target nucleic acids form a group of at least two different target nucleic acids, wherein the method should avoid the troublesome use of beads, be cost effective, avoid steps which lead to loss of rarely found nucleic acids and have the advantage of being easy to perform, preferentially with fewer steps involved then when making use of hybridization techniques.

It is further an objective of the invention to provide for uses and kits for the method according to the invention.

### SUMMARY OF THE INVENTION

The problem described above was solved with a method for the preferential nucleic acid synthesis reaction of one or more selected regions of one or more target nucleic acids form a group of at least two different target nucleic acids comprising the steps of combining in a reaction mixture, at least two different target nucleic acid molecules with at least on nucleotide triphosphate, a polymerase, a buffer and at least one primer additionally, combining the at least two different target nucleic acid molecules with at least one blocking agent, which is capable of binding at least one of the nucleic acid template molecules in a sequence specific manner, in such a way that the polymerase is not able to utilize the bound target nucleic acid molecule as a template and, exposing the reaction mixture to a temperature at which the at least one amplification primer may bind its template and exposing the reaction mixture to a temperature at which the polymerase is capable of elongating the at least one amplification primer. Thus, in summary the invention in its broadest sense makes use of a blocking agent which preferentially binds those templates which are not desirable when amplifying the nucleic acids.

### DETAILED DESCRIPTION OF THE PREFERRED EMBODIMENTS

As outlined above, the inventors have found that combining in a reaction mixture, at least two different target nucleic acid molecules with at least one nucleotide triphosphate, a polymerase, a buffer and at least one primer additionally, combining the at least two different target nucleic acid molecules with at least one blocking agent, which is capable of binding at least one of the nucleic acid template molecules in a sequence specific manner, in such a way that the polymerase is not able to utilize the bound target nucleic acid molecule as a template and, exposing the reaction mixture to a temperature at which the at least one amplification primer may bind its template and exposing the reaction mixture to a temperature at which the polymerase is capable of elongating the at least one amplification primer, enables a method for the preferential nucleic acid synthesis reaction of one or more selected regions of one or more target nucleic acids form a group of at least two different target nucleic acids.

In this method according to the invention it is preferred that the at least one primer according to the invention comprises preferably between 3 and 100 nucleotides more preferably between 10 and 50 nucleotide most preferably between 12 and 25 nucleotides. It is important that the at least one primer is of sufficient length to bind the desired at least two target nucleic acid molecules with sufficient stringency. PNA-DNA hybrid oligonucleotides (see Finn, P. J. et al., N.A.R. 24, 3357-3363 (1996), Koch, T. et al., Tetrahedron Letters 36, 6933-6936 (1995), Stetsenko, D. A. et al., Tetrahedron letters 37, 3571-3574 (1996), Bergmann, F et al., Tetrahedron Letters 36 6823-6826 (1995) and Will, D. W. et al., Tetrahedron 51,12069-12082 (1995)) are also considered as primers for the process of the present invention.

As a thermally stable polymerase, a DNA polymerase may be selected from the group comprising Taq DNA polymerase, Tth DNA polymerase or Klentaq (Taq DNA polymerase (-exo5'-3'), Korolev et al., (1995) Proc. Natl. Acad. Sci. USA 92, 9246-9268. The use of Taq DNA polymerase in the method of the present invention is especially preferred. One skilled in the art will recognize that numerous different thermally stable polymerases may be used, such as Pfu DNA Polymerase, Pwo DNA Polymeras may be used (Dabrowski S and Kur J, Protein Expr. Purif. 14,131-138 (1998), "Cloning and expression in Escherichia coli of the recombinant histagged DNA polymerases from Pyrococcus furiosus and Pyrococcus woesei"). When working with RNA other polymerases are preferred, namely those that have reverse transcriptase activity such as Taq polymerase under certain conditions or Tth polymerase.

Alternatively, as a thermally stable polymerase, a DNA polymerase which has a decreased discrimination against the four ddNTPs with respect to wild-type Taq DNA polymerase in the buffer or under the conditions used for thermal cycling is preferred. More preferably, a DNA polymerase Taq polymerase carrying a "Tabor-Richardson" mutation or a functional derivative thereof which also lacks 5'-3' exonuclease activity such as, for example, AmplitaqFS™ (Taq DNA polymerase (-exo5'-3')(F667Y), Tabor and Richardson (1995), loc. cit.), Taquenase™ (Taq DNA polymerase Δ235(-exo5'-3')(F667Y), Tabor and Richardson (1995), loc. cit.) and ThermoSequenase™ (Taq DNA polymerase (-exo5'-3')(F667Y), Tabor and Richardson (1995), loc. cit.) as well as mixtures thereof or other DNA polymerases and mixtures thereof which are thermally stable can be used in the process of the present invention. The use of Thermo Sequenase™ or any other DNA polymerase having a high ability to incorperate ddNTPs in the method of the present invention may be preferred depending on the use.

Alternatively as a thermally stable polymerase, a DNA polymerase which has a decreased discrimination against labeled nucleotides may be used such polymerases are known in the art.

The present invention, *i.e.* the process also provides for the use of two or more polymerases in the process or additional enzymes such as amplification enhancing reagents such as thermally stable pyrophosphatase or enzymes which enhance the processivity of the polymerase such as PCNA (proliferating cell nuclear antigen) homologues. Enzyme mixtures may be equally applied.

The number of thermal cycles may range from about 1 to about 50 depending on the amount of template DNA and its purity.

Routinely, cycling consists of (i) a denaturing cycle, (ii) an annealing cycle and (iii) an extension cycle. Alternatively, only one or two cycles may be applied, (i) a denaturing cycle and (ii) an annealing and extension cycle. At least the primer should be able to bind and a temperature should be applied where the polymerase may elongate the bound at least one primer.

Preferably the denaturing cycle is performed at between 100°C and 85°C, more preferably at between 98°C and 90°C, most preferably at between 96°C and 92°C.

Preferably the annealing cycle is performed at between 80°C and 45°C, more preferably at between 70°C and 50°C, most preferably at between 60°C and 55°C.

Preferably the extension cycle is performed at between 80°C and 50°C, more preferably at between 75°C and 60°C, most preferably at between 73°C and 68°C.

Preferably the denaturing cycle is performed for 3 minutes, more preferably for 30 seconds, most preferably for under 10 seconds.

Preferably the annealing cycle is performed for 3 minutes, more preferably for 1 minute, most preferably for under 30 seconds.

Preferably the extension cycle is performed for 3 minutes, more preferably for 90 seconds, most preferably for under 30 seconds, however the extension time may vary depending on the length of the template, in particular the extension time may be raised if the template length increases.

Buffers components which can be used can include, but are not limited to, Tris-HCI at a pH of about 7.0 to 10 and concentration of about 2 to 60 mM, ammonium sulfate at a concentration of about 10-20 mM, preferably 15 mM, MgCl₂ at a concentration of about 1 to 10 mM, and optionally, about 0.05 mM mercaptoethanol, about 0.28% Tween® 20 and/or about 0.02% Nonidet® 40.

The preferred buffer for Pwo (Roche Molecular Biochemicals) is 10 mM Tris-HCI, pH 8.85, 25 mM KCI, 5mM ammonium sulfate, 2mM magnesium sulfate; optimal Mg2+ concentration is between 1-10 mM.

When performing the method according to the invention on RNA, e.g. SuperScript™ One-Step RT-PCR with Platinum Taq (Fa. GibcoBRL/Invitrogen may be used.

Nucleotide triphosphates are preferably deoxynucleotides and can be selected from, but are not limited to, dGTP, dATP, dTTP and dCTP. In addition, derivatives of deoxynucleotides, which are defined as those deoxynucleotides which are capable of being incorperated by a thermally stable polymerase into nascent nucleic acid molecules synthesized in the thermal cycling reaction, can also be used according to the invention. Such derivatives include, but are not limited to thionucleotides, 7-deaza-2'-dGTP, 7-deaza-2'-dATP as well as deoxyinosine triphosphate which may also be used as a replacement deoxynucleotide for dATP, dGTP, dTTP or dCTP. The above mentioned deoxynucleotides and derivatives thereof are preferably used at a concentration of about 200 µM to about 2,5 mM when amplifying DNA and 200 µM when amplifying RNA.

Preferably the nucleotides are mixes of all four nucleotides A, C, G, and T and at 2,5 µM each.

In one embodiment one or more of the nucleotides incorporated are labeled. For example, single and differential labels may consist of the group comprising enzymes such as β-galactosidase, alkaline phosphatase and peroxidase, enzyme substrates, coenzymes, dyes, chromophores, fluorescent, chemiluminescent and bioluminescent labels such as FITC, Cy5, Cy5.5, Cy7, Texas-Red and IRD40(Chen et al. (1993), J. Chromatog. A 652: 355-360 and Kambara et al. (1992), Electrophoresis 13: 542-546), ligands or haptens such as biotin, and radioactive isotopes such as ³H, ³⁵S, ³²P ¹²⁵I and 14C.

In one embodiment of the method of the invention, the nucleic acid molecule to be amplified can be present in the form of total genomic DNA, which is preferably in an uncloned or unpurified form. Generally all forms of template may be used, *e.g.* purified nucleic acids, *i.e.* nucleic acids where one fraction may be enriched or not, one example being plasmid DNA the other purified genomic DNA. The process may be suited for use with complex mixtures of DNA such being purified but not substantially fractionated genomic DNA or non-complex mixtures such being purified and substantially fractionated DNA e.g. plasmid DNA.

In a further preferred embodiment of the method of the invention, the nucleic acid molecule to be amplified can be present in the form of RNA. One polymerase or a mixture of two polymerases maybe utilized: a first DNA polymerase for example, Taq polymerase, and a second DNA polymerase with the capability to reverse transcribe RNA into DNA preferably Taq DNA polymerase (Jones et al., Nucl. Acids Res. 17: 8387-8388 (1989)) or Tth DNA polymerase (Myers et al., Biochemistry 30: 7666-7672 (1991)).

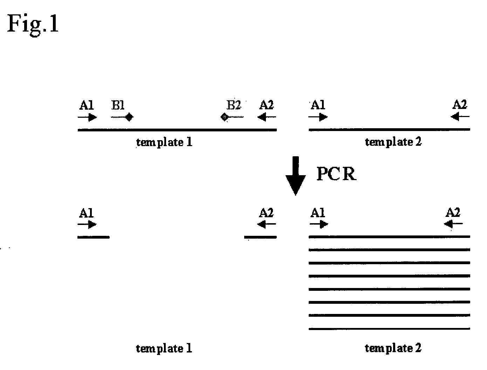

When speaking of the one blocking agent, which is capable of binding at least one of the nucleic acid template molecules in a sequence specific manner, in such a way that the polymerase is not able to utilize the bound target nucleic acid molecule as a template it is mean that e.g a proteinor another chemical entity is able to bind specifically to that or those templates that are not desired. This may be in the region where the at least one amplification primer binds or 3'-prime to that location (see Fig. 1)

The blocking agent may be for example an antibody, a nucleic acid of any kind such as DNA, RNA, PNA or the like.

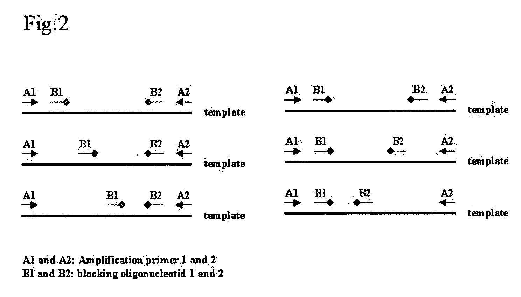

It is essential that the blocking agent is able to bind with sufficient specificity as well as hinder the polymerase from making use of the template that is not desired. The latter may be done at any position 3'-prime to the location of the primer or at the site of the primer (see Fig. 2).

In one embodiment of the invention the method is carried our using RNA as a template and the polymerase present has the capability to reverse transcribe RNA into DNA. Alternatively the method may be performed on DNA. Mixture may work likewise.

In a preferred embodiment the method further comprising at least a second amplification primer which is capable of binding the complementary strand of the strand that the first amplification primer binds. Thus, the method may be performed with at least two primers present. There is no limitation to the number of primers that may be present.

It is preferred that the blocking agent binds 3'-prime to at least one of the amplification primers present in the reaction. One may use more than one blocking agent and these may bind at various locations on various templates. The inventors also have shown that the different blocking agents may be used at various molar amounts, thereby suppressing the undesired templates to different degrees.

The inventors have shown that is particularly preferable if the blocking agent is a nucleic acid molecule comprising a nucleic acid sequence which is sufficiently complementary to the target nucleic acid molecule in order for it to bind the undesired template and which can not be elongated by a polymerase at its 3' end. Thus, where *e.g.* most DNA molecules have a 3'-OH which may serve as site for binding a nucleotide triphosphate it is preferred in the method according to the invention that the nucleic acid blocking agent caries a modified 3'-prime end which does not permit the polymerase to add nucleotides. One embodiment for accomplishing this is if the blocking agent carries a nucleotide at its 3'-end which has no OH-group at the C-3 position of the ribose. Other embodiments comprise solutions whereby the 3'-end carries a modification which hinders the polymerase from elongating the nucleic acid. The blocking nucleic acid molecule may carry for example a 3' modification selected from the group comprising phosphate group, amino group, biotin group or a terminally inverted 3' end nucleotide (often designated: 3'-3"').

The inventors can show that it is advantageous if the blocking nucleic acid molecule carries a 5' modification which prohibits the polymerase from either 5' exonucleolytic attack on the blocking agent or strand displacement thereof. Preferred solutions for accomplishing this are, e.g. that the 5' modification is a modification selected from the group comprising phosphate group and/or amino group.

It is advantageous if the blocking nucleic acid molecule is present in molar excess of any other primer present in the reaction. It is preferred if the blocking nucleic acid is present in the reaction at a molar ratio of about 1 to 1 to about 100 to 1 in excess of the amplification primers. It is particularly preferred if the ratio is about 10 to 1. Here the ratio refers to the molar amount of one blocking nucleic acid to one primer.

The inventors have found that the method works particularly well if the polymerase that is used lacks 5'-3' exonuclease activity and/or strand displacement capability. In a preferred embodiment such polymerases may be chosen from the group comprising Pwo DNA polymerase and/or Pfu DNA Polymerase.

The method is particularly suited for the creation of DNA libraries. In this application libraries may be created which are more homogenous with respect to the distribution of the different genes or clones present. Example 2 shows how the present invention is able to create libraries which are superior to libraries created with methods known in the art.

The invention may also be used to create kits for the preferential nucleic acid synthesis reaction of one or more selected regions of one or more target nucleic acids form a group of at least two different target nucleic acids comprising, one or more amplification primers and, a blocking agent that is capable of binding at least one of the nucleic acid template molecules present in a sequence specific manner, in such a way that a polymerase is not able to utilize the bound target nucleic acid molecule as a template.

### EXAMPLES:

### Example 1:

Method according to the invention using plasmid DNA. Three different plasmids sharing the same modified pBluescript SKII plasmid backbone but different inserts in their multiple cloning site were chosen. All of these posses the same binding sites for amplification primer NPCR1 - 5' tcg agc ggc cgc ccg ggc agg t 3' (SEQ ID NO. 1) and NPCR2 '5' agc gtg gtc gcg gcc gag gt 3' (SEQ ID NO. 2) however flanking different insertions. The three plasmids were chosen in a way that the fragments corresponding to the different plasmids could be distinguished by their size when amplified with the two primers mentioned above (fragment 1 = 570bp; fragment 2 = 450bp; fragment 3 = 400bp).

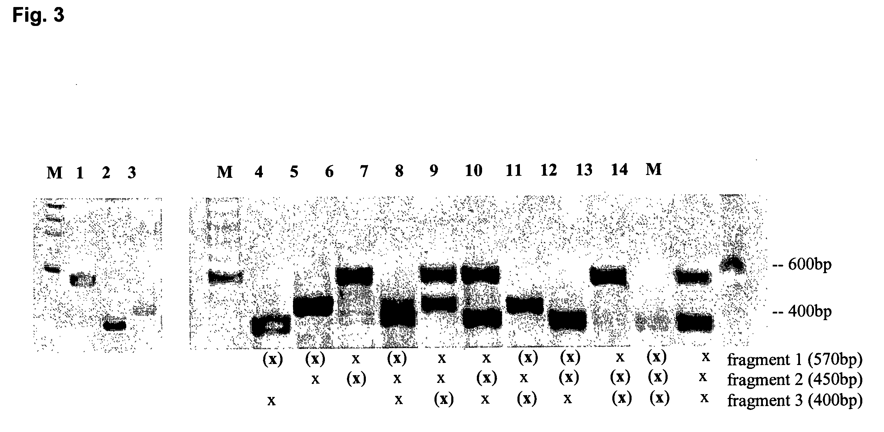

A control PCR reaction was performed to confirm the predicted size of the amplification products (Fig. 3; lane 1-3). The reaction was carried out using 0.1 ng Plasmid DNA as template, 2 units Pwo DNA polymerase (Roche Molecular Biochemicals), 1 µl of the primer NPCR1 and NPCR2 (10 µM), 10 µl dNTP solution (1 mM each). Double distilled Water was added up to a final reaction volume of 50 µl. Thermal cycling was performed in the DNA thermal cycler (GeneAmp PCR System 9700; Perkin Elmer). The temperature cycling parameters were as follows: 1 initial denaturation step at 75°C for 5 minutes, followed by 30 seconds at 94°C; 30 cycles of 10 seconds at 94°C, 30 seconds at 60°C, 90 seconds at 72°C; and a final elongation step of 5 minutes at 72°C.

In a second step two of the three plasmids were mixed in equal molar ratios. A PCR was performed using this mixture as a template. The conditions above were used with the exception that two blocking agents according to the invention (1 µl of a 100 µM solution, each; see below for nucleic acid sequence details) were added to suppress the amplification of one of the two plasmid-templates specifically (Fig. 3; lanes 4-6). The blocking oligonucleotides were specifically designed to align nested to the amplification primers. They each have an annealing temperature of 70-72°C. To avoid the elongation of these oligonucleotides by the DNA-Polymerase the 3' end of the oligonucleotide was protected by an aminolink (C7). The protection of the 3' end worked equally by adding a phosphate group.

In all the three tested combinations (Fig. 3; lane 4 to 6) a normal amplification of the non suppressed template took place while the amplification of the suppressed one was drastically decreased.

To confirm these results all the three templates were mixed in an equal molar ratio and PCR experiments were performed under the conditions mentioned above (Fig. 3; lane 7 - 13). This time the inventors attempted to suppress template one (lane 7 - 9), template two (lane 10-12) or even all the three (lane 13) templates used. To suppress templates specifically, two blocking oligonucleotides (1 µl of a 100µM solution; showing the same characteristics mentioned above) were added for each of them. Again, a specific suppression was observed while the other template(s) remained unaffected. The results of lane 10 to 13 indicate clearly that it is possible to suppress more than 66% and up to nearly 100% of a possible template using the method according to the invention.

### The following blocking agents were applied:

Fragment 1 (Mm.20407) was suppressed using the following two oligonucleotides: "blocking oligonucleotide 1": 5'- ggg act tgg tgg tca cac agc agc agc ctc - 3' (SEQ ID No. 3), "blocking oligonucleotide 2": 5'- agg gct ctc tgc agc cag gta ctt ctc acg tc - 3' (SEQ ID No. 4);

Fragment 3 (Mm. 25377) was suppressed using the following two oligonucleotides "blocking oligonucleotide 3": 5'-ggt tgc cta aat ggg gct aag gag aag aaa ggg g-3' (SEQ ID No. 5), "blocking oligonucleotide 4": 5' - gct gag gga cac tgg gct cag agg ttt cct g - 3' (SEQ ID No. 6);

Fragment 2 (Mm.34374) was suppressed using the following two oligonucleotides "blocking oligonucleotide 5" : 5' - ttg tag atg cac gga ggg gga gga aga gga - 3' (SEQ ID No. 7) and "blocking oligonucleotide 6" : 5'- cca gtg gcg tct ctt cct gca tgg gtg ac 3' (SEQ ID No. 8).

### Example 2:

### Construction of cDNA libraries using the method according to the invention

In order to demonstrate the power of the invention for suppressing certain templates and thus its particular suitability for library construction mouse pancreas mRNA was used to prepare several cDNA libraries. The pancreatic RNA was chosen as it is to be expected that many copies of proteolytic enzymes would be present which pose a particularly difficult task in depleting.

The mRNA was initially reverse transcribed using the cDNA synthesis system (Gibco Life Technolgies) and a PolyT-Primer carrying a biotin residue at the 5' end (5' - atg atg ctg gag ttt ttt ttt ttt ttt ttt vn - 3' (SEQ ID No. 9) with N = A,G,C or T and V = A, G or C). The cDNA was sonicated for 1 minute using the Misonix 2020 system (Fa. Misonix Inc.) with a puls setting of 0.9. The resulting DNA fragments were "blunted", i.e. blunt ends were created, using Pwo DNA-Polymerase (Roche). The 3'-ends of the cDNAs were bound to streptavidin-paramagnetic beads following the user manual (Fa. Dynal). A doublestranded adapter oligonucleotide (adapter oligo1: 5' - 5'-cta ata cga ctc act ata ggg cgc gcc agc gtg gtc gcg gcc gag gt - 3' (SEQ ID No. 10), adapter oligo2: 5' - acc tcg gcc gcg ac - 3' (SEQ ID No. 11 was ligated to the 5'-end of the bound DNA. The reaction was carried out over night at 16°C in a 20 µl volume using 1 µl of the bound DNA, 2 µl T4-DNA Ligase (40u/µl; Roche) and 5 µl of the doublestranded adaptor DNA.

To achieve a sufficient amount of material for the subsequent steps a PCR amplification of the 3'ends was carried out using 10 µl of the ligation reaction as template DNA, 2u Pwo DNA Polymerase (Roche), 1 µl of the SAPCR-1 primer (5' - c taa tac gac tca cta tag ggc - 3' (SEQ ID No. 14) and SAPCR-2 and (Biotin-atg atg ctg gag ttt ttt ttt ttt ttt t-3' (SEQ ID No. 15) primer (10 µM each) and 10 µl dNTP solution (1 mM each). Bidestilled Water was added to a final reaction volume of 50µl. While a control reaction was performed without any additional "blocking oligonucleotides" three parallel reactions were performed adding the following 8 oligonucleotides to the PCR reaction (1µl of a 100µM solution each):
Blocking oligonucleotide 1: GGG ACT TGG TGG TCA CAC AGC AGC AGC CTC - 3' (SEQ ID No. 3), Blocking oligonucleotide 2: AGG GCT CTC TGC AGC CAG GTA CTT CTC ACG TC- 3' (SEQ ID No. 4), Blocking oligonucleotide 3: GGT TGC CTA AAT GGG GCT AAG GAG AAG AAA GGG G- 3' (SEQ ID No. 5), Blocking oligonucleotide 4: GCT GAG GGA CAC TGG GCT CAG AGG TTT CCT G- 3' (SEQ ID No. 6), Blocking oligonucleotide 5: TTG TAG ATG CAC GGA GGG GGA GGA AGA GGA- 3' (SEQ ID No. 7), Blocking oligonucleotide 6: CCA GTG GCG TCT CTT CCT GCA TGG GTG AC- 3' (SEQ ID No. 8), Blocking oligonucleotide 7: CCC CAC CTT TGC CCC AGA TAC CAA GAC ACC- 3' (SEQ ID No. 9), Blocking oligonucleotide 8: GGA GTC CAG TGG CCT CCC CAA GAT GGC TC- 3' (SEQ ID No.10)

The blocking oligonucleotides 1 and 2 were designed to suppress amplification of the pancreatic lipase related protein 1 (Mm.20407, designating the cluster ID of the mouse UniGene database at NCBI); oligonucleotides 3 and 4 to suppress amplification of the carboxypeptidase precursor (Mm.25377 designating the cluster ID of the mouse UniGene database at NCBI), oligonucleotides 5 and 6 to suppress amplification of the chymotrypsin B precursor (Mm.34374 designating the cluster ID of the mouse UniGene database at NCBI), oligonucleotides 7 and 8 to suppress amplification of the P6-5 gene (Mm.2131 designating the cluster ID of the mouse UniGene database at NCBI).

All oligonucleotides posses an annealing temperature of 70-72°C. To avoid the elongation of these nucleotids by the DNA-Polymerase the 3' end of the oligonucleotid was protected by an aminolink (at the C7 position).

Thermal cycling was performed in the DNA Thermal Cycler (GeneAmp PCR System 9700; Perkin Elmer). The temperature cycling parameters were as follows: 1 initial denaturation step at 75°C for 5 minutes, followed by 30 seconds at 94°C; 30 cycles of 10 seconds at 94°C, 30 seconds at 60°C, 90 seconds at 72°C; and a final elongation step of 5 minutes at 72°C.

After amplification the material all four attempts (the control and the three suppression reactions) were treated the same. After purification of the PCR reaction with the QIAquick PCR Purification Kit (Qiagen) the DNA was released from the streptavidin beads by digesting the material with Bpml enzyme (NEB). The digested material was purified by agarose gel electrophoresis and subsequent QIAquick Gel extraction kit (Qiagen). In the following step a double stranded adapter oligonucleotide (adapter oligonucleotide 3: 5' - tcg agc ggc cgc ccg ggc agg ttt - 3' (SEQ ID NO. 12), adapter oligonucleotide 4: 5' - acc tgc ccg ggc ggc cgc - 3' (SEQ ID NO. 13) was ligated to the 3'-end of the eluted DNA. The reaction was carried out over night at 16°C in a 100 µl volume using the eluted DNA (about 30 µl), 2µl T4-DNA Ligase (400 u/µl; Roche) and 20 µl of the double stranded adapter DNA (10 uM). The ligation product was digested with Asc I (NEB) and unidirectional cloned in an predigested Asc I, Xho I vector system (modified pBluescript derivative). The DNA was transformed into competent DH10B cells (Gibco). About 150 clones of the three different approaches were sequenced and the number of clones determined corresponding to the four most frequent transcripts in mouse pancreas.

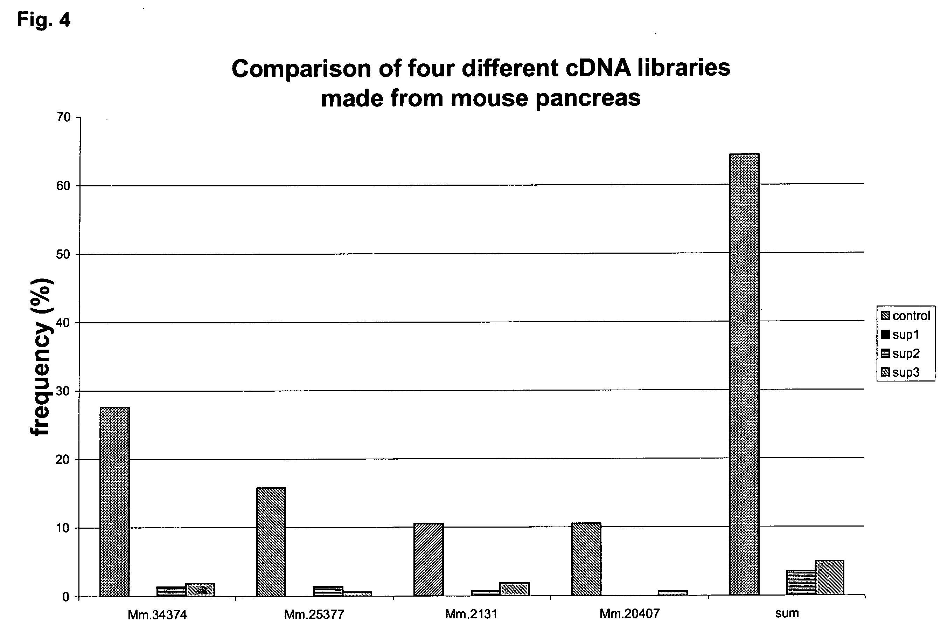

While in the reference approach using the protocol without the addition of "blocking oligonucleotides" in the PCR reaction the number of clones referring to these 4 most abundant transcripts in mouse pancreas (Mm. 20407, Mm. 25377, Mm. 34374, Mm. 2131) were about 65%, this number decreased drastically down to less than 5% in the two approaches where the method according to the invention was applied (see also Fig. 4).

### FIGURE CAPTIONS

Fig. 1: Schematic representation of the method according to the invention :
   Figure one depicts a schematic representation of one embodiment of the method according to the invention. In this example the two templates are present in the reaction, template 1 and template 2. The preferential amplification of template 2 is desired. In addition to the amplification primers A1 and A2 two blocking agents are present in the reaction. In this case these are B1 and B2 which are blocking nucleic acids. The blocking nucleic acids may not be elongated by the polymerase which is present in the reaction as the respective 3-prime ends are blocked (diamond shape). After amplification template 2 has been amplified and is abundantly present.
Fig. 2: Method according to the invention used on plasmid DNA:
   Figure two depicts a schematic representation of further embodiments of the method. The blocking agent may be located at various positions and may be specific for different templates.
Fig. 3: Method according to the invention used on plasmid DNA:
   In lanes 1 to 3 the two amplification primers were added to one single plasmid DNA as positive controls for the PCR reaction (for details see examples). In lane 4 to 6 two different plasmid DNA species were used as templates for the amplification, one of these templates was specifically suppressed using the method according to the invention by addition of a pair of blocking nucleic acid agents having the capability to bind both strands of one plasmid DNA species and located nested to the amplification primers.
   A mixture of three templates was used in lanes 7 to 14. In addition to the two amplification primers at least one pair of blocking oligonucleotides was added. Fragments which are suppressed in their amplification are designated with an "(x)", while non suppressed products are indicated with "x". Lanes containing DNA ladders are designated with "M"
Fig. 4: Construction of cDNA libraries making use of the method according to the invention:
   Figure four depicts graphically the results obtained in Experiment 2.

SEQUENCE LISTING

<110> LION Bioscience AG

<120> METHOD FOR THE PREFERENTIAL NUCLEIC ACID SYNTHESIS REACTION OF ONE OR MOR
E SELECTED REGIONS OF ONE OR MORE TARGET NUCLEIC ACIDS

<130> L-0046-00-EP-01

<160> 15

<170> PatentIn version 3.0

<210> 1
<211> 22
<212> DNA
<213> Artificial

<400> 1
tcgagcggcc gcccgggcag gt 22

<210> 2
<211> 20
<212> DNA
<213> Artificial

<400> 2
agcgtggtcg cggccgaggt 20

<210> 3
<211> 30
<212> DNA
<213> Artificial

<400> 3
gggacttggt ggtcacacag cagcagcctc 30

<210> 4
<211> 32
<212> DNA

Seite 1

<213>  Artificial

<400>  4
agggctctct gcagccaggt acttctcacg tc                                    32


<210>  5
<211>  34
<212>  DNA
<213>  Artificial

<400>  5
ggttgcctaa atggggctaa ggagaagaaa gggg                                  34


<210>  6
<211>  31
<212>  DNA
<213>  Artificial

<400>  6
gctgagggac actgggctca gaggtttcct g                                     31


<210>  7
<211>  30
<212>  DNA
<213>  Artificial

<400>  7
ttgtagatgc acggaggggg aggaagagga                                       30


<210>  8
<211>  29
<212>  DNA
<213>  Artificial

<400>  8
ccagtggcgt ctcttcctgc atgggtgac                                        29


<210>  9

Seite 2

<211>  32
<212>  DNA
<213>  Artificial

<220>
<221>  misc_feature
<222>  (32)..(32)
<223>  May be nucleotide A, C, G or T


<220>
<221>  misc_feature
<222>  (31)..(31)
<223>  May be nucleotide A, C or G not T.


<400>  9
atgatgctgg agtttttttt tttttttttt vn                                    32


<210>  10
<211>  47
<212>  DNA
<213>  Artificial

<400>  10
ctaatacgac tcactatagg gcgcgccagc gtggtcgcgg ccgaggt                    47


<210>  11
<211>  14
<212>  DNA
<213>  Artificial

<400>  11
acctcggccg cgac                                                        14


<210>  12
<211>  24
<212>  DNA
<213>  Artificial

Seite 3

<400>  12
tcgagcggcc gcccgggcag gttt                                              24

<210>  13
<211>  18
<212>  DNA
<213>  Artificial

<400>  13
acctgcccgg gcggccgc                                                     18

<210>  14
<211>  22
<212>  DNA
<213>  Artificial

<400>  14
ctaatacgac tcactatagg gc                                                22

<210>  15
<211>  28
<212>  DNA
<213>  Artificial

<400>  15
atgatgctgg agtttttttt tttttttt                                          28

Seite 4

## Claims

1. Method for the preferential nucleic acid synthesis reaction of one or more selected regions of one or more target nucleic acids form a group of at least two different target nucleic acids comprising the steps of combining in a reaction mixture
a) at least two different target nucleic acid molecules with at least one nucleotide triphosphate, a polymerase, a buffer and at least one primer;
b) additionally, combining the at least two different target nucleic acid molecules with at least one blocking agent, which is capable of binding at least one of the nucleic acid template molecules in a sequence specific manner, in such a way that the polymerase is not able to utilize the bound target nucleic acid molecule as a template and,
c) exposing the reaction mixture to a temperature at which the at least one amplification primer may bind its template and exposing the reaction mixture to a temperature at which the polymerase is capable of elongating the at least one amplification primer.

2. Method according to claim 1,
**characterized in that** the nucleic acid template is RNA and the polymerase present has the capability to reverse transcribe RNA into DNA.

3. Method according to claim 1,
**characterized in that** the nucleic acid template is DNA.

4. Method according to claim 3,
further comprising at least a second amplification primer which is capable of binding the complementary strand of the strand that the first amplification primer binds.

5. Method according to any of the above claims,
**characterized in that** the blocking agent binds 3''-prime to at least one of the amplification primers present in the reaction.

6. Method according to claim 5,
**characterized in that** the blocking agent is a nucleic acid molecule comprising a nucleic acid sequence which is sufficiently complementary to the target nucleic acid molecule in order for it to bind and which can not be elongated by a polymerase at its 3' end.

7. Method according to claim 6,
**characterized in that** the blocking nucleic acid molecule carries a 5' modification which prohibits the polymerase from either 5' exonucleolytic attack on the blocking agent or strand displacement thereof.

8. Method according to claim 7,
**characterized in that** the 5' modification is a modification selected from the group comprising a phosphate group and/or an amino group.

9. Method according to claim 6 to 8,
**characterized in that** the blocking nucleic acid molecule carries a 3' modification selected from the group comprising a phosphate group, an amino group, a biotin group, a nucleotide lacking an -OH group at the C-3 position of the ribose and/or a terminally inverted 3' end nucleotide.

10. Method according to any of claims 6 to 9,
**characterized in that** the blocking nucleic acid is present in the reaction at a molar ratio of about 1 to 1 to about 100 to 1 in excess of the amplification primers.

11. Method according to any of claims 6 to 10,
**characterized in that** the polymerase lacks 5'-3' exonuclease activity and/or strand displacement capability.

12. Method according to claim 11,
**characterized in that** the polymerase is chosen from the group comprising Pwo DNA polymerase and/or Pfu DNA Polymerase.

13. Use of the method according to any of the claims 1 to 12 for the creation of DNA libraries.

14. Kit for the preferential nucleic acid synthesis reaction of one or more
selected regions of one or more target nucleic acids form a group of at least two different target nucleic acids comprising,
a) one or more amplification primers and
b) a blocking agent that is capable of binding at least one of the nucleic acid template molecules present in a sequence specific manner, in such a way that a polymerase is not able to utilize the bound target nucleic acid molecule as a template.
